# EUROPEAN PATENT APPLICATION

(11) **EP 1 582 584 A1**
(43) Date of publication of application: **05.10.2005**
(21) Application number: 03768192.1
(22) Date of filing: 25.12.2003
(51) Int. Cl.: C12N 9/12, C07H 21/02, C12P 19/34, C12N 15/54, C12N 1/21

(54) **PROCESS FOR PRODUCING PNPase**

(30) Priority: 26.12.2002 JP 2002376780
(71) Applicant: Nippon Shinyaku Co., Ltd., Kyoto 601-8550 (JP)
(72) Inventor: MURAI, Masatoshi, Ashiya-shi, Hyogo 659-0033 (JP)
(74) Representative: Vossius & Partner
(86) International application number: PCT/JP2003/016653
(87) International publication number: WO 2004/058959

(57) **Abstract**

The invention provides a process for producing PNPase, wherein PNPase can be produced easily with high efficiency, and problematic contamination with endotoxin in synthesis of a nucleic acid polymer as a raw material of pharmaceutical preparations can be reduced. PNPase is produced by*Escherichia coli* or the like having a T7 RNA polymerase gene, transformed with an expression vector having a PNPase gene and a T7 promoter ligated therein. For further facilitating the step of purifying PNPase, an expression vector having a tag gene is utilized and the culture time is prolonged.

## Description

### Technical Field

The present invention relates to a process for producing PNPase (polynucleotide phosphorylase) which is an enzyme useful for producing a synthetic nucleic acid polymer.

### Background Art

PNPase is an enzyme found in 1955 by S. Ochoa, and catalyzes reversible polymerization of ribonucleoside diphosphate to release inorganic phosphorus. This enzyme is distributed widely in bacterias but does not occur in animals.

This enzyme is useful in synthesis of a high-molecular homopolymer or copolymer or an oligomer having a determined sequence because ribonucleoside diphosphate can be polymerized by the action of this enzyme *in vitro*.

PNPase can be obtained classically by separation and extraction from bacterias, but a method capable of producing it in a large amount by recombinant DNA techniques is also known as shown in US Patent No. 4912496. This patent discloses a method which involves integrating a PNPase gene (also referred to hereinafter as "pnp gene") into a vector containing a suitable expression-regulating signal to increase the amount of the enzyme gene expressed, accumulating PNPase in a large amount in a bacteria transformed with the vector, then disrupting the bacteria, and extracting and purifying the PNPase.

A T7 RNA polymerase (Genbank registered number M38308) specifically and highly efficiently promotes the transcription of a gene downstream of a T7 promoter (US Patent Nos. 4912496, 5693489 and 5869320).

### Disclosure of Invention

A main object of the present invention is to provide a process for producing PNPase, which is capable of producing PNPase more easily with higher efficiency than by conventionally known processes, and reducing problematic contamination with endotoxin in synthesis of a nucleic acid polymer as a raw material of pharmaceutical preparations.

The present inventors made extensive study, and as a result, they found that *Escherichia coli* or the like having a T7 RNA polymerase gene, transformed with an expression vector having the pnp gene and a T7 promoter ligated therein, can be used to solve the problem described above, and the present invention was completed.

The present invention includes, for example:
(1) a process for producing PNPase, comprising at least the following steps:
   A. a step of constructing an expression vector comprising a prokaryote-derived PNPase gene integrated into a plasmid having a T7 promoter as an expression-regulating signal;
   B. a step of transforming *Escherichia coli* or its analogous bacteria having a T7 RNA polymerase gene using the expression vector;
   C. a step of allowing the resulting transformant to express the PNPase gene thereby accumulating PNPase in the bacteria; and
   D. a step of recovering the bacteria having PNPase accumulated therein, and extracting and purifying the PNPase, or
(2) a process for producing PNPase, comprising at least the following steps:
   A. a step of constructing an expression vector comprising a prokaryote-derived PNPase gene integrated into a plasmid having a T7 promoter as an expression-regulating signal;
   B. a step of transforming *Escherichia coli* or its analogous bacteria having a T7 RNA polymerase gene using the expression vector;
   C'. a step of allowing the resulting transformant to express the PNPase gene thereby accumulating PNPase in the bacteria, and further continuing to allow expression until the bacteria is disrupted to release the PNPase into a supernatant outside of the bacteria; and
   D'. a step of recovering and purifying the PNPase released in the supernatant.

In the above processes, the process (2) is preferable.

An origin of the pnp gene includes, but is not limited to, *Escherichia coli* (for example, K12 strain, 0157 strain) and its analogous bacteria (for example, *Salmonella typhimurium*). In the present invention, a pnp gene derived from *Escherichia coli* (particularly, K12 strain) is preferable.

Although the plasmid having a T7 promoter as an expression-regulating signal is not particularly limited insofar as it is a plasmid having a T7 promoter, the plasmid is preferably a plasmid vector replicable in a bacteria and having a specific restriction enzyme cleavage site and high copy number in the bacteria. Specific examples include pET plasmids (Novagen), pRSET-A, p-RSET-B and pRSET-C (Invitrogen).

The plasmid preferably has a tag gene by which a tag can be added to the PNPase according to the present invention (also referred to hereinafter as the enzyme). The tag gene includes, for example, a His tag gene, T7 tag gene, S tag gene, Nus tag gene, GST tag gene, DsbA tag gene, DsbC tag gene, CBD_{cex} tag gene, CBD_{cenA} tag gene, CBD_{clos} tag gene, Trx tag gene, HSV tag gene, and 3×FLAG tag gene. Particularly the His tag gene is suitable.

*Escherichia coli* or its analogous bacteria as the host is not particularly limited insofar as it has a T7 RNA polymerase gene, but a bacteria used in a recombinant DNA experiment is preferable. Specific examples include *Escherichia coli* BL21 [DE3], *Escherichia coli* BL21 [DE3] pLysS, *Escherichia coli* BLR [DE3], *Escherichia coli* Rosetta [DE3], and *Escherichia coli* B834 [DE3].

The enzyme produced according to the present invention can be used to synthesize various nucleic acid polymers such as nucleic acid homopolymer, nucleic acid copolymer and oligo nucleic acid. Specific examples of the synthesizable nucleic acid polymers include polyinosinic acid, polycytidylic acid, polyuridylic acid, polyadenylic acid, polyguanylic acid, poly(5-bromocytidylic acid); poly(2-thiocytidylic acid), poly(7-deazainosinic acid), poly(2'-azidoinosinic acid), poly(cytidine-5'-thiophosphoric acid), poly(1-vinylcytidylic acid), poly(cytidylic acid, uridylic acid), poly(cytidylic acid, 4-thiouridylic acid), and poly(adenylic acid, uridylic acid).

Every procedure for carrying out the present invention can be conducted by methods known in the art.

### I. With respect to steps A to D

### Step A:

For example, the pnp gene can be cloned in a usual manner from chromosomal DNA in *E*. *coli.* Specifically, cloning by colony hybridization can be mentioned.

By polymerase chain reaction (PCR), an NdeI cleavage site is then introduced into an initiation codon of the pnp gene, while an EcoRI cleavage site is introduced downstream of a termination codon of the gene, and a pnp gene-containing DNA fragment from the NdeI cleavage site to the EcoRI cleavage site can be obtained in a usual manner.

This DNA fragment is then mixed with, and ligated to, a plasmid having a T7 promoter, cleaved previously with NdeI and EcoRI, whereby the objective expression vector can be constructed.

### Step B :

The expression vector obtained as described above can be used in a usual manner to transform *E. coli* having a T7 RNA polymerase gene or its analogous bacteria. The transformed *E. coli* etc. can be freeze-stored in a usual manner.

The transformation method can be carried out in a usual manner, and is not particularly limited. Specifically, methods such as calcium chloride method, electroporation method etc. can be mentioned.

### Step C or C':

The transformant can grow by cultivation in a usual manner in a medium where it can grow. Prior to cultivation for growth, the transformant is preferably pre-cultured for example overnight at 37°C. Then, cultivation is initiated until suitable turbidity (for example a turbidity of 0.4 to 1.0 at 600 nm) is reached, and then the pnp gene can be expressed by adding a suitable amount of a suitable expression inducer to induce the enzyme in the bacteria. After the inducer is added, cultivation is conducted for example for 7 to 9 hours, whereby the accumulation of the enzyme in the bacteria is usually maximized, and cultivation is further continued for example for additional 24 hours, usually the bacteria can be autonomously digested to release the enzyme into a culture supernatant. When the enzyme is released into a culture supernatant, a step of disrupting the bacteria and an extraction step are not necessary, and thus the enzyme can be obtained in high yield with less contamination with endotoxin.

The expression inducer includes, for example, isopropyl-β-D-thiogalactopyranoside (referred to hereinafter as IPTG) and lactose.

Cultivation of the transformant can be carried out in a usual manner in a medium containing nutrition sources such as carbon source, nitrogen source etc. necessary for growth of the bacteria. As the medium, a medium used in conventional culture of *Escherichia coli,* such as 2×YT medium, LB medium, M9CA medium etc., can be used. Cultivation can be effected for example at a culture temperature of 20 to 40°C, if necessary under aeration and stirring. For preventing elimination of the plasmid during cultivation, a suitable antibiotic (ampicilin, kanamycin or the like depending on a chemical resistance marker in the plasmid) can be added in a suitable amount to the culture solution to culture the bacteria. To prevent overflow caused by foaming in a later stage of cultivation, a suitable defoaming agent (for example, Adecanol LG-109 (manufactured by Asahi Denka Kogyo K.K.), Antifoam AF Emulsion (manufactured by Nacalai Tesque)) can be added in a suitable amount.

### Step D or D':

The method of extracting and purifying the enzyme from the bacteria recovered after the cultivation and induction can be carried out in a usual manner.

When the enzyme is accumulated in the bacteria, the bacteria is suspended in a suitable buffer and disrupted physically by a method such as sonication or with a French press, and microbial residues are removed, whereby the enzyme can be obtained. If purification is necessary, the enzyme can be purified by salting-out with ammonium sulfate, subsequent dialysis, treatment with a solvent such as ethanol, various kinds of chromatographic techniques, and ultrafiltration.

When the enzyme is released in a culture supernatant through cultivation and induction for a long time, the step of disrupting the bacteria as described above can be omitted.

In the case of the enzyme expressed with a tag, the enzyme can be recovered and purified more easily in a usual manner. For example, the enzyme can be purified by treating the recovered supernatant with a column suitable for the added tag.

The enzyme produced by the process of the present invention can also be treated with a column for removing endotoxin in order to synthesis an endotoxin-free nucleic acid polymer usable as a pharmaceutical preparation. When the enzyme is produced by releasing it into a culture supernatant through cultivation and induction for a long time, the step of disrupting the bacteria is unnecessary, thus eliminating the contamination with endotoxin which can be caused upon disruption.

### II. Method of synthesizing a nucleic acid polymer

A nucleic acid polymer can be synthesized by allowing the enzyme obtained in the process of the present invention to act in a usual manner on ribonucleoside diphosphate. The enzyme with a tag can be used as it is, but may be used after removing the tag in a usual manner.

### Brief Description of Drawings

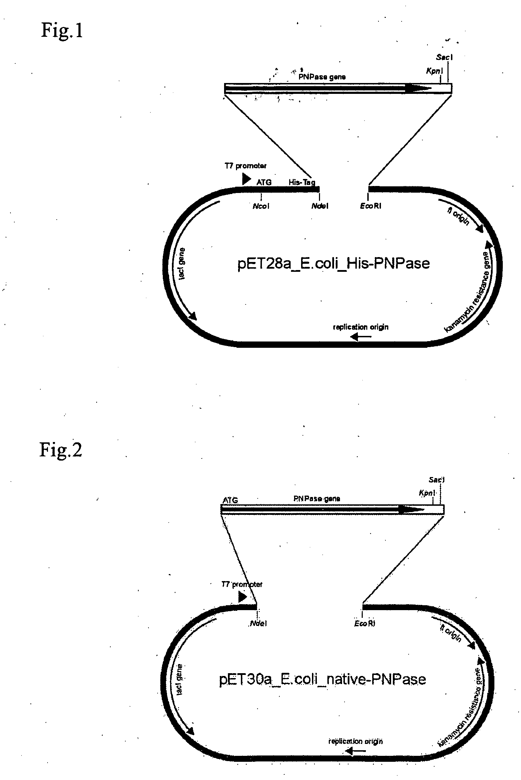

Fig. 1 shows a plasmid map of His tag-added PNPase (His-PNPase) expression plasmid pET28a*_E*. *coli*_His-PNPase.
Fig. 2 shows a plasmid map of His tag-free PNPase (native PNPase) expression plasmid pET30a_*E*._*coli*_native PNPase.

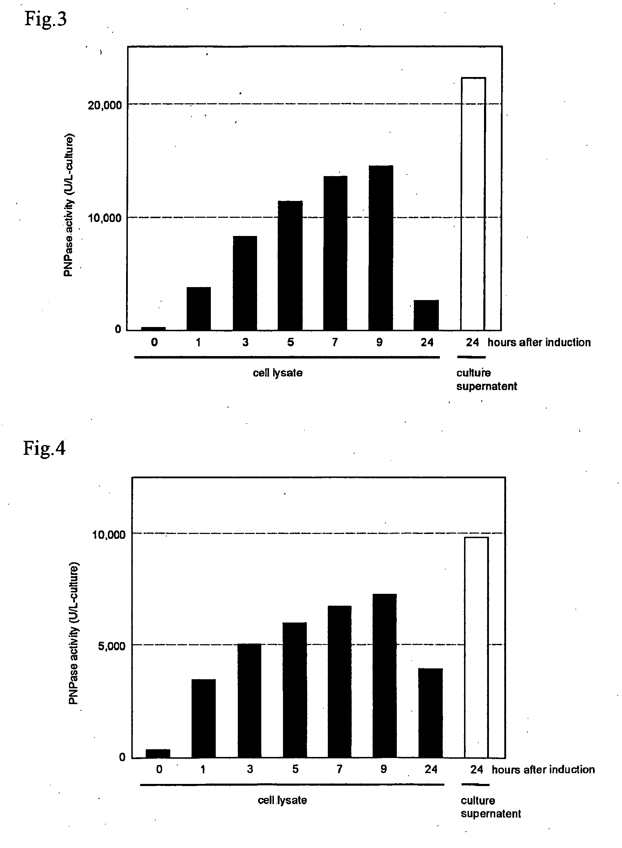

Fig. 3 shows the activity of His tag-added PNPase. The activity of PNPase (U/L culture solution) is shown on the ordinate, and the culture time (hours) after induction of expression is shown on the abscissa. The black column shows the activity of PNPase in the disrupted microbial solution, and the white column shows the activity of PNPase in the culture supernatant.
Fig. 4 shows the activity of His tag-free PNPase (i.e. PNPase to which a His tag had not been added). The activity of PNPase (U/L culture solution) is shown on the ordinate, and the culture time (hours) after induction of expression is shown on the abscissa. The black column shows the activity of PNPase in the disrupted microbial solution, and the white column shows the activity of PNPase in the culture supernatant.

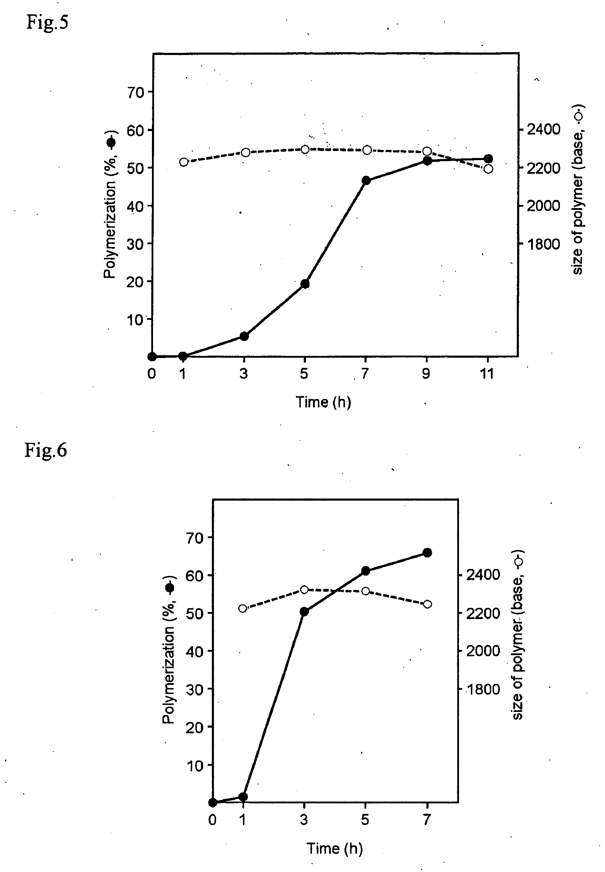

Fig. 5 shows the synthesis reaction yield and average chain length of polyinosinic acid. The synthesis reaction yield (%) is shown on the left ordinate, the average chain length (number of bases) on the right ordinate, and the time (hours) on the abscissa. A change in synthesis reaction yield is shown by ―●― and a change in average chain length by ... ○ ....
Fig. 6 shows the synthesis reaction yield and average chain length of polycytidylic acid. The synthesis reaction yield (%) is shown on the left ordinate, the average chain length (number of bases) on the right ordinate, and the time (hours) on the abscissa. A change in synthesis reaction yield is shown by ―●― and a change in average chain length by ···○···

### Best Mode for Carrying Out the Invention

Hereinafter, the present invention is described in more detail by reference to the Examples and Test Example. As a matter of course, the present invention is not limited to the following examples.

### Example 1

### (1) Construction of an expression vector in which the pnp gene was integrated

By colony hybridization, the pnp gene was cloned from chromosomal DNA in *Escherichia coli* C600K, and by PCR, an NdeI cleavage site was introduced into an initiation codon of the pnp gene, and an EcoRI cleavage site was introduced downstream of a termination codon of the gene, and a pnp gene-containing DNA fragment from the NdeI cleavage site to the EcoRI cleavage site was obtained in a usual manner.

This DNA fragment was then mixed with, and ligated to, an expression vector plasmid pET28a (containing a His tag gene, manufactured by Novagen) having a dephosphorylated 5'-end, cleaved previously with NdeI and EcoRI, whereby an expression vector having the tag gene was constructed.

This expression vector was composed of pET28a DNA having the DNA fragment of about 2400 base pairs inserted into it, and this plasmid was designated pET28a_*E*. *coli*_His-PNPase (see Fig. 1). As a result of decoding of the whole DNA sequence of the pnp gene, including a region derived from the vector, the region derived from the vector agreed with a sequence published by Novagen, and the region of the pnp gene agreed completely with a DNA sequence corresponding to the pnp gene of *E*. *coli* K12 described in Genbank registered number NC_000913 in a public gene database.

The pET28a_*E*. *coli*_His-PNPase DNA was cleaved with NdeI and EcoRI and then subjected to agarose gel electrophoresis to extract the NdeI-EcoRI DNA fragment of about 2400 base pairs. Then, this DNA fragment was mixed with, and ligated to, an NdeI- and EcoRI-cleaved expression vector plasmid pET30a whose 5'-end was dephosphorylated (not containing a His tag gene, manufactured by Novagen), whereby a tag gene-free expression vector was constructed.

This expression vector was composed of pET30a DNA having the DNA fragment of about 2400 base pairs inserted into it, and this plasmid was designated pET30a_*E*. *coli*_native-PNPase (see Fig. 2).

### (2) Preparation of transformants

The plasmid pET28a_*E*. *coli_*His-PNPase or pET30a_*E*. *coli*_native-PNPase was used in a usual manner to transform *Escherichia coli* BL21 [DE3] (manufactured by Novagen) to prepare each transformant.

### (3) Production of the enzyme

The transformant of *E. coli* BL21 [DE3] containing pET28a_*E*. *coli*_His-PNPase or the transformant of *E*. *coli* BL21 [DE3] containing pET30a_*E*. *coli*_native-PNPase was pre-cultured by shake culture at 37°C for about 16 hours (MR-200L shake culture machine, manufactured by Takasaki Kagaku) in a kanamycin-containing modified terrific broth medium (24 g/L yeast extract (manufactured by Nacalai Tesque), 12 g/L trypton (manufactured by Nacalai Tesque), 0.4% [v/v] glycerol).

LB medium (LB BROTH BASE, Cat No. 12780-052, manufactured by Invitrogen) was introduced into a 10-L portable jar fermenter (LS-10, manufactured by Oriental Yeast Co., Ltd.), and the above culture was inoculated into it (turbidity at 600 nm was about 0.2 when cultivation was initiated) and cultured at 37°C, 1 wm, 500 rpm, under aeration. When the turbidity at 600 nm reached 0.5 to 0.7, IPTG (manufactured by Nacalai Tesque) was added in an amount of 0.4 mM to induce expression thereby producing each of the 2 enzymes.

To prevent elimination of the expression vector, kanamycin was added at a concentration of 25 mg/L. As a defoaming agent, Adecanol LG-109 (Asahi Denka Kogyo K.K.) was added in an amount of about 0.2 mL every 7 L of the medium.

### (4) Recovery, extraction, purification

① First, the enzyme with a His tag was purified from the bacteria in 112 L culture (7-L culture×16 times) collected 3 hours after induction of expression with IPTG
The bacteria was suspended in extraction buffer A (20 mM Tris-HCl, pH 8.0, 0.5 M sodium chloride, 10% glycerol) in an amount of about 1/60 relative to the culture, and after addition of egg-white lysozyme at 50 mg/L, the bacteria was shaken at room temperature for 30 minutes and then frozen at -80°C. The frozen bacteria was rapidly thawed at 37°C and then disrupted for about 5 minutes by sonication at the maximum power with sonication cell disrupter XL2020 and a disrupting hone (Cat No. 200) manufactured by Astrason. The disrupted bacteria was centrifuged at 20,000×g at 4°C for 60 minutes, and a supernatant was collected to prepare 1.6 L crude microbial extract. The crude microbial extract was subjected to Ni⁺ affinity chromatography (φ2.6×20, His Bind Flactogel M, manufactured by Novagen) in the following manner to purify the His tag-added enzyme. The crude microbial extract was applied at 5 mL/min. onto the column equilibrated with extraction buffer A, and the resin was washed with 1 L extraction buffer A, and the His tag-added enzyme was finally eluted from the column by extraction buffer A containing 1 L of 0.5 M imidazole. Then, diafiltration using a ultrafiltration membrane was conducted for the purpose of changing pH and removing sodium chloride and imidazole. 1 L eluate containing the enzyme was concentrated to about 600 mL with a ultrafiltration cartridge (PREP/SCALE-TFF, fractionation molecular weight of 10,000, manufactured by Millipore), and then subjected to ultrafiltration while the amount of the enzyme solution was kept constant by adding a buffer (50 mM Tris-HCl, pH 7.0, 0.15 M sodium chloride, 5% glycerol). The ultrafiltration was continued until the filtrate reached 7 L, and the composition of the buffer in the enzyme solution was thus changed. Then, this enzyme solution was applied onto an endotoxin-eliminating column (Kurimover II, φ2.6×10 cm, manufactured by Kurita Water Industries Ltd.). The enzyme solution was passed at 1.7 mL/min. through the activated Kurimover II column, and the passing fractions were collected. Then, diafiltration using a ultrafiltration cartridge was conducted for the purpose of changing pH and removing sodium chloride. While the amount of the enzyme solution was kept constant by adding a buffer (20 mM Tris-HCl, pH 8.0, 5% glycerol), ultrafiltration was conducted. The ultrafiltration was continued until the filtrate reached 7 L so that the composition of the buffer in the enzyme solution was changed, and then the enzyme solution was freeze-stored at -20°C. A sample was collected in each step of purification and measured for its enzyme activity and endotoxin level.
The results are shown in Table 1.

**Table 1**

| | *PNPase* | | | | Endotoxin level (EU/mL) | Endotoxin/PN Pase (EU/U) |
|---|---|---|---|---|---|---|
| | Activity (U/mL), | Volume (mL) | Total activity (U) | Yield (%) | | |
| Crude extract | 553 | 1,600 | 885,520 | 100 | ND | ND |
| Ni⁺ affinity column eluate | 520 | 1,000 | 520,378 | 59 | ND | ND |
| First diafiltration | 365 | 1,000 | 364,825 | 41 | 626,345 | 1,717 |
| Kurimover II column eluate | 239 | 950 | 227,124 | 26 | 868 | 3.6 |
| Second diafiltration | 248 | 800 | 198,714 | 22 | 2,299 | 9.3 |
| ND: not measured | | | | | | |

As is evident from Table 1, about 200,000 units of the enzyme could be obtained from the bacteria in the 112 L microbial culture (cultivation for 3 hours after induction). Almost all the endotoxin contained in a large amount after the first diafiltration was removed by the Kurimover II column treatment, and only 9.3 EU endotoxin remained per unit of PNPase in the final product.
② Then, the His tag-added enzyme was purified from the bacteria in 56 L culture (7L×8 times) collected 7 hours after induction of expression with IPTG The bacteria was suspended in extraction buffer B (20 mM Tris-HCl, pH 8.0, 0.5 M sodium chloride, 5% glycerol) in an amount of about 1/30 relative to the culture, and after addition of egg-white lysozyme at 50 mg/L, the bacteria was shaken at room temperature for 30 minutes and then frozen at -80°C. The frozen bacteria was rapidly thawed at 37°C and then disrupted for about 5 minutes by sonication at the maximum power with sonication cell disrupter XL2020 and a disrupting hone (Cat No. 200) manufactured by Astrason. The disrupted bacteria was centrifuged at 20,000×g at 4°C for 60 minutes, and a supernatant was collected to prepare 1.5 L crude microbial extract. The crude microbial extract was subjected to Ni⁺ affinity chromatography in the following manner to purify the His tag-added enzyme. The crude microbial extract was applied at 5 mL/min. onto the column equilibrated with extraction buffer B, and the resin was washed with 1 L extraction buffer B, and the His tag-added protein was finally eluted from the column by 1 L extraction buffer B containing 0.5 M imidazole. Then, diafiltration using a ultrafiltration membrane was conducted for the purpose of changing pH and removing sodium chloride and imidazole. 1 L eluate containing the enzyme was concentrated to about 600 mL with the ultrafiltration cartridge, and then subjected to ultrafiltration while the amount of the enzyme solution was kept constant by adding a buffer (50 mM Tris-HCl, pH 7.0, 0.15 M sodium chloride, 5 mM magnesium chloride, 5% glycerol). The ultrafiltration was continued until the filtrate reached 7 L, and the composition of the buffer in the enzyme solution was thus changed. Then, this enzyme solution was applied onto a Kurimover II column. The enzyme solution was applied at 1.7 mL/min. onto the activated Kurimover II column, and the passing fractions were collected. Then, diafiltration using a ultrafiltration cartridge was conducted for the purpose of changing pH and removing sodium chloride. While the amount of the enzyme solution was kept constant by adding a buffer (20 mM Tris-HCl, pH 8.0, 5 mM magnesium chloride, 5% glycerol), ultrafiltration was conducted. The ultrafiltration was continued until the filtrate reached 7 L so that the composition of the buffer in the enzyme solution was changed, and then the enzyme solution was freeze-stored at -20°C. A sample was collected in each step of purification and measured for its enzyme activity and endotoxin level.
The results are shown in Table 2.

**Table 2**

| | *PNPase* | | | | Endotoxin level (EU/mL) | Endotoxin/ PNPase (EU/U) |
|---|---|---|---|---|---|---|
| | Activity (U/mL) | Volume (mL) | Total activity (U) | Yield (%) | | |
| Crude extract | 615 | 1,500 | 921,808 | 100 | ND | ND |
| Ni⁺ affinity column eluate | 301 | 1,100 | 330,740 | 36 | ND | ND |
| First diafiltration | 288 | 1,100 | 253,377 | 34 | 156,387 | 543 |
| Kurimover II column eluate | 230 | 1,100 | 227,124 | 27 | 8,180 | 35.5 |
| Second diafiltration | 213 | 800 | 170,771 | 19 | 207 | 1 |
| ND: not measured | | | | | | |

As is evident from Table 2, about 170,000 units of the enzyme could be obtained from the bacteria in the 56 L culture (cultivation for 7 hours after induction). This amount is almost the same as the amount of the enzyme purified from the bacteria in the 112 L culture collected 3 hours after induction, thus proving that the yield of the enzyme was increased by prolonging the culture time. Almost all the endotoxin contained in a large amount after the first diafiltration was removed by the Kurimover II column treatment, and only 1.0 EU endotoxin was contained per unit of the enzyme in the final product. This level was lower than the endotoxin level (9.3 EU/U-PNPase) contained in the enzyme purified from the bacteria in the 112 L culture collected 3 hours after induction.
③ Then, the His tag-added enzyme was purified from a culture supernatant from 28 L culture (7L culture×4 times) collected 24 hours after induction of expression with IPTG The culture supernatant was applied at a rate of 150 mL/min. onto an anion-exchange column (QAE-TOYOPERL 550C, φ140×70 mm) previously equilibrated with 20 mM Tris-HCl, pH 8.0. The column was washed with 5 L of 20 mM Tris-HCl buffer, pH 8.0, containing 0.1 M sodium chloride, and then the enzyme adsorbed on the ion-exchange resin was eluted with 5 L of 20 mM Tris-HCl buffer, pH 8.0, containing 0.5 M sodium chloride, to give a crude enzyme solution. The crude enzyme solution was subjected to Ni⁺ affinity chromatography to purify the His tag-added enzyme. The crude enzyme solution was applied at 5 mL/min. onto the column equilibrated with extraction buffer B, and the resin was washed with 1 L extraction buffer B and then with 1 L extraction buffer B containing 50 mM imidazole, and the His tag-added enzyme was finally eluted from the column by 0.5 L extraction buffer B containing 0.5 M imidazole. Then, diafiltration using a ultrafiltration membrane was conducted for the purpose of changing pH and removing sodium chloride and imidazole. 1 L eluate containing the enzyme was concentrated to about 500 mL with a ultrafiltration cartridge (PREP/SCALE-TFF, fractionation molecular weight of 30,000, manufactured by Millipore), and then subjected to ultrafiltration while the amount of the enzyme solution was kept constant by adding a buffer (50 mM Tris-HCl, pH 7.0, 0.15 M sodium chloride). The ultrafiltration was continued until the filtrate reached 7 L, and the composition of the buffer in the enzyme solution was thus changed. Then, this enzyme solution was applied onto a Kurimover II column. The enzyme solution was treated at 1.7 mL/min. with the activated Kurimover II column, and the passing fractions were collected. Then, diafiltration using a ultrafiltration membrane (PREP/SCALE-TFF, fractionation molecular weight of 30,000, manufactured by Millipore) was conducted for the purpose of changing pH and removing sodium chloride. While the amount of the enzyme solution was kept constant by adding a buffer (20 mM Tris-HCl, pH 8.0, 5 mM magnesium chloride, 5% glycerol), ultrafiltration was conducted. The ultrafiltration was continued until the filtrate reached 7 L so that the composition of the buffer in the enzyme solution was changed, and then the enzyme solution was freeze-stored at -20°C. A sample was collected in each step of purification and measured for its enzyme activity and endotoxin level.

The results are shown in Table 3.

**Table 3**

| | *PNPase* | | | | Endotoxin level (EU/mL) | Endotoxin/P NPase (EU/U) |
|---|---|---|---|---|---|---|
| | Activity (U/mL) | Volume (mL) | Total activity (U) | Yield (%) | | |
| Crude extract | 23.9 | 28,000 | 669,698 | 100 | ND | ND |
| Anion-exchange column eluate | 70.4 | 5,000 | 352,029 | 53 | ND | ND |
| Ni⁺ affinity column eluate | 158 | 500 | 79,057 | 12 | 7,811,004 | 49,437 |
| First diafiltration | 135 | 500 | 67,500 | 10 | ND | ND |
| Kurimover II column eluate | 111 | 500 | 55,300 | 8 | 22 | 0.2 |
| Second diafiltration | 87.5 | 500 | 43, 742 | 7 | 105 | 1.2 |
| ND: not measured | | | | | | |

As is evident from Table 3, about 50,000 units of the enzyme could be obtained from the 28 L culture supernatant (cultivation for 24 hours after induction). In this enzyme, the presence of other proteins was hardly recognized by protein purity assay with SDS-PAGE/Coomassie blue staining. Almost all the endotoxin contained in a large amount after the first diafiltration was removed by the Kurimover II column treatment, and only 1.2 EU endotoxin was contained per unit of the enzyme in the final product. This level was lower than the endotoxin level (9.3 EU/U-PNPase) contained in the enzyme purified from the bacteria in the 112 L culture collected 3 hours after induction. Accordingly, the purification of the enzyme from the culture supernatant can be a method capable of omitting steps (e.g. microbial disruption) whose scale-up is difficult and giving the enzyme of high purity.

### Test Example 1. Measurement of the activity of the enzyme

0, 1, 2, 3, 5, 7, 9 and 24 hours after induction of expression (that is, after addition of IPTG), the sample was collected and the activity of the enzyme was measured.

The results indicated that as shown in Figs. 3 and 4, the accumulation of both the His tag-added enzyme and the His tag-free enzyme was maximized in the bacterias 7 to 9 hours after induction, and was reduced 24 hours after induction. 24 hours after induction, the enzyme in an amount higher than the enzyme accumulated in the bacteria 7 to 9 hours after induction had been released into the culture supernatant (see Figs. 3 and 4).

### ① Preparation of samples for measuring the activity of the enzyme

400 mL culture solution was collected in a 500-mL centrifugal tube and centrifuged at 5,000×g, room temperature for 5 minutes (SCR-20BA manufactured by Hitachi Koki), to recover the bacteria. The supernatant was stored as culture supernatant. The bacteria was suspended in 30 mL buffer (20 mM Tris-HCl, pH 8.0, 0.15 M sodium chloride, 10% [v/v] glycerol, 1 mM Tris-carboxyethylphosphine HCl) containing 50 mg/L egg-white lysozyme, then left at room temperature for 15 minutes and stored at -80°C. Freezing/thawing was conducted twice to disrupt the *Escherichia coli* mildly which was then disrupted for about 30 seconds by sonication at the maximum power with sonication cell disrupter XL2020 and a disrupting hone (Cat No. 200) manufactured by Astrason. The disrupted bacteria was centrifuged at 10,000×g at 4°C for 10 minutes, and a supernatant was collected to prepare a crude microbial extract.

### ② Measurement of the activity of the enzyme

20 µL enzyme solution and 80 µL reaction solution for the enzyme (125 mM Tris-HCl, pH 9.0, 0.25 mg/mL bovine serum albumin, 0.5 mM EDTA·2Na, 6 mM magnesium chloride, 25 mM adenosine diphosphate trisodium) were added to a 1.5-mL centrifugal tube (manufactured by Eppendorf), mixed gently and kept at 37°C for 15 minutes. The reaction was terminated by adding 0.9 mL of ice-cold 4% aqueous sodium perchlorate, and then the reaction solution was left on ice for 10 minutes. The reaction solution was centrifuged at 4°C at 15,000 rpm for 5 minutes (MR-150 manufactured by TOMY SEIKO Co., Ltd.) to separate a supernatant. For quantifying inorganic phosphate released into the reaction supernatant, 50 µL of the supernatant and 50 µL Tassky-Shorr reagent (0.5 M sulfuric acid, 10 g/L ammonium molybdate, 50 g/L ferrous sulfate) were added to each well of a 96-well plate (manufactured by Corning), then shaken for 30 seconds and left at room temperature for 5 minutes. The absorbance at 660 nm was measured (Model 550, manufactured by Bio-Rad) to calculate the activity of the enzyme. 1 U defined herein refers to the amount of the enzyme by which 1 µmol inorganic phosphate is released by reaction at 37°C, pH 9.0, for 15 minutes.

### Example 2. Synthesis of polyinosinic acid

Using the enzyme purified from the bacteria in the 112 L culture, polyinosinic acid (RNA homopolymer) was synthesized. A small-scale synthesis reaction was previously conducted to determine conditions under which the polymer having a long chain length on average could be synthesized with high reaction yield. The synthesis of polyinosinic acid was conducted at 37°C in a reaction solution having a total volume of 350 mL with a composition (100 mM 2-[4-(2-hydroxyethyl)-1-piperazinyl]ethanesulfonic acid (HEPES)-NaOH, pH 7.5, 0.4 mM EDTA·2Na, 50 mM magnesium chloride, 0.1 g/L inosine diphosphate·3Na (manufactured by Yamasa Corporation), 11.43 U/mL His-PNPase). A sample was collected with time, and a part of the sample was analyzed by gel filtration HPLC under denaturing conditions (that is, in the presence of 7 M urea), and the average chain length and reaction yield were calculated.

The chain length was determined by using, as indicators, products of pUC 119 (manufactured by Takara Shuzo Co., Ltd.) decomposed with restriction enzymes EcoRI, NarI and NspI (manufactured by New England Bio Lab).

The results are shown in Fig. 5. As is evident from Fig. 5, polyinosinic acid having an average chain length of about 2200 bases was obtained with a reaction yield of about 50% by the reaction at 37°C for 11 hours.

### Example 3. Synthesis of polycytidylic acid

Polycytidylic acid was synthesized in an analogous manner. The synthesis of polycytidylic acid was conducted at 37°C in a reaction solution having a total volume of 350 mL with a composition (100 mM glycine-NaOH, pH 9.0, 0.4 mM EDTA·2Na, 25 mM magnesium chloride, 0.1 g/L cytidine diphosphate·3Na (manufactured by Yamasa Corporation), 11.43 U/mL His-PNPase). A sample was collected with time, and a part of the sample was analyzed by gel filtration HPLC under denaturing conditions, and the average chain length and reaction yield were calculated.

The results are shown in Fig. 6. As is evident from Fig. 6, polycytidylic acid having an average chain length of about 2200 bases was obtained with a reaction yield of about 65% by the reaction at 37°C for 7 hours.

### Industrial Applicability

According to the present invention, the enzyme with a tag can be expressed to make purification very easy, and further there is brought about an unexpected effect that the amount of the enzyme produced in *Escherichia coli* is increased about 2-fold. By devising the culture method, the enzyme can be purified rapidly and easily even in large-scale culture, by releasing the enzyme, without accumulation in the bacteria, into a culture supernatant while preventing the contamination with a large amount of endotoxin caused upon disruption of the bacteria.

## Claims

1. A process for producing PNPase, comprising at least the following steps:
(A) a step of constructing an expression vector comprising a prokaryote-derived PNPase gene integrated into a plasmid having a T7 promoter as an expression-regulating signal;
(B) a step of transforming *Escherichia coli* or its analogous bacteria having a T7 RNA polymerase gene using the expression vector;
(C) a step of allowing the resulting transformant to express the PNPase gene thereby accumulating PNPase in the bacteria; and
(D) a step of recovering the bacteria having PNPase accumulated therein, and extracting and purifying the PNPase.

2. The process according to claim 1, wherein the steps (C) and (D) are the following steps (C') and (D') respectively:
(C') a step of allowing the transformant to express the PNPase gene thereby accumulating PNPase in the bacteria, and further continuing to allow expression until the bacteria is disrupted to release the PNPase into a supernatant outside of the bacteria; and
(D') a step of recovering and purifying the PNPase released in the supernatant.

3. The process according to claim 1 or 2, wherein the plasmid has a tag gene capable of adding a tag to the PNPase to be produced.

4. The process according to claim 3, wherein the tag gene is a His tag gene, T7 tag gene, S tag gene, Nus tag gene, GST tag gene, DsbA tag gene, DsbC tag gene, CBD_{cex} tag gene, CBD_{cenA} tag gene, CBD_{clos} tag gene, Trx tag gene, HSV tag gene, or 3×FLAG tag gene.

5. The process according to any one of claims 1 to 4, wherein the prokaryote is *Escherichia coli.*

6. The process according to claim 5, wherein the *Escherichia coli* is *Escherichia coli* K12 or *Escherichia coli* O157.

7. The process according to any one of claims 1 to 6, wherein the *Escherichia coli* having a T7 RNA polymerase gene is *Escherichia coli* BL21 [DE3], *Escherichia coli* BL21 [DE3] pLysS, *Escherichia coli* BLR [DE3], *Escherichia coli* Rosetta [DE3], or *Escherichia coli* B834 [DE3].

8. A synthetic nucleic acid polymer produced by using PNPase produced by the process of any one of claims 1 to 7.

9. The synthetic nucleic acid polymer according to claim 8, wherein the synthetic nucleic acid polymer is polyinosinic acid, polycytidylic acid, polyuridylic acid, polyadenylic acid, polyguanylic acid, poly(5-bromocytidylic acid), poly(2-thiocytidylic acid), poly(7-deazainosinic acid), poly(2'-azidoinosinic acid), poly(cytidine-5'-thiophosphoric acid), poly(l-vinylcytidylic acid), poly(cytidylic acid, uridylic acid), poly(cytidylic acid, 4-thiouridylic acid), or poly(adenylic acid, uridylic acid).
